# EUROPÄISCHE PATENTANMELDUNG

(11) **EP 2 357 473 A1**
(43) Veröffentlichungstag der Anmeldung: **17.08.2011**
(21) Anmeldenummer: 10151689.6
(22) Anmeldetag: 26.01.2010
(51) Int. Cl.: G01N 33/487, A61B 5/00, B65H 20/18, A61B 5/151, G01N 35/00

(54) **Bandsystem zur Probenuntersuchung**

(71) Anmelder: F. Hoffmann-La Roche AG, 4070 Basel (CH); Roche Diagnostics GmbH, 68305 Mannheim (DE)
(72) Erfinder: Harttig, Herbert, Dr., 67434 Neustadt (DE)
(74) Vertreter: Pfiz, Thomas

(57) **Zusammenfassung**

Die Erfindung betrifft ein Bandsystem zur Probenuntersuchung mit einem Verbrauchsmittel (16) tragenden Trägerband (14), einem Bandspeicher (12) zum Aufnehmen des Trägerbandes (14) und einer Transportvorrichtung (20) zum sukzessiven Bereitstellen der Verbrauchsmittel (16) unter Vorschub des Trägerbandes (14) in einer Bandlaufrichtung. Erfindungsgemäß wird vorgeschlagen, dass die Transportvorrichtung (20) zwei mit dem Trägerband (14) in Eingriff bringbare Bandschieber (24) aufweist, und dass die Bandschieber (24) durch einen oszillierenden Vorschubantrieb (22) wechselweise in Bandlaufrichtung vorwärts und zurück bewegbar sind.

## Beschreibung

Die Erfindung betrifft ein Bandsystem zur Probenuntersuchung, insbesondere von Körperflüssigkeiten in einem Handgerät, mit einem vorzugsweise als Stech- und/oder Nachweiselemente ausgebildete Verbrauchsmittel tragenden Trägerband, einem Bandspeicher zum Aufnehmen des Trägerbandes und einer Transportvorrichtung zum sukzessiven Bereitstellen der Verbrauchsmittel in einer Gebrauchsposition unter Vorschub des Trägerbandes in einer Bandlaufrichtung.

Analytische Kleingeräte, welche ein Testband umfassen, das gleichsam als Magazin eine Vielzahl diagnostischer Testelemente für Einmaltests trägt, sind beispielsweise in der DE-A 10343896 beschrieben. Bei solchen auch aus der Praxis bekannten Ausführungen ist der Weitertransport des Bandes durch das Aufwickeln auf einen zentral angetriebenen Bandwickel vorgesehen. Das Aufwickeln erfordert einen kräftigen Antrieb, da das Drehmoment an einem Wickel mit zunehmendem Wickeldurchmesser zunimmt. Um einen Wickelantrieb in einem ausreichend engen Belastungsbereich betreiben zu können, darf der Durchmesser und damit das Drehmoment bei nahezu leerem und bei nahezu vollem Wickel nicht zu unterschiedlich sein. Dies kann dadurch erreicht werden, dass ein ausreichend groß dimensionierter Wickelkern verwendet wird, was jedoch zu erhöhtem Platzbedarf führt.

Aus der US 2007/189928 ist ein Testgerät für Körperflüssigkeiten mit einer Bandkassette bekannt, die durch einen Benutzer über einen Fortschaltmechanismus betätigt werden kann. Hierzu ist in einer Ausführung ein manueller Antrieb über einen Druckknopf und einen flexiblen Arm sowie einen daran angelenkten Reibfinger vorgesehen. Am Ende einer Fortschaltbewegung wird der Finger vom Band abgehoben und durch Federrückholung wieder in eine Ausgangsposition gebracht. Das Band befindet sich somit beim Abheben des Fingers in einem spannungsarmen Zustand, d.h. bei Zugspannung kann das Band zurückgleiten und beim Anlaufen gegen einen Widerstand kann es sich selbst zurückschieben. Nachteilig ist weiterhin, dass nur ein intermittierender Betrieb möglich ist, und dass eine definierte Transportstrecke nur erreicht wird, wenn Indexmarken in das Band eingebracht werden.

Ausgehend hiervon liegt der Erfindung die Aufgabe zugrunde, die im Stand der Technik bekannten Bandsysteme hinsichtlich des Bandtransportes weiter zu verbessern, wobei mit begrenztem Bauaufwand ein kompakter und exakt arbeitender Bandtransporteur geschaffen werden soll.

Zur Lösung dieser Aufgabe wird die im Patentanspruch 1 angegebene Merkmalskombination vorgeschlagen. Vorteilhafte Ausgestaltungen und Weiterbildungen der Erfindung ergeben sich aus den abhängigen Ansprüchen.

Die Erfindung geht von dem Gedanken aus, eine gleichmäßiege Drehbewegung über eine periodisch veränderliche Linearbewegung kontinuierlich auf das Band zu übertragen. Dementsprechend wird erfindungsgemäß vorgeschlagen, dass die Transportvorrichtung zwei mit dem Trägerband in Eingriff bringbare Bandschieber aufweist, und dass die Bandschieber durch einen motorgetriebenen oszillierenden Vorschubantrieb wechselweise in Bandlaufrichtung vorwärts und zurück bewegbar sind. Damit ist ein kontinuierlicher Bandtransport möglich, so dass Applikationspositionen exakt angefahren werden können, ohne dass ein Benutzer manuell eingreifen muss. Durch den Schiebetransport kann auf Bandwickel verzichtet werden, wobei auch Transportwiderstände zuverlässig überwunden werden können, insbesondere auch bei dünnem biegeschlaffem Bandmaterial und unterschiedlichster Bandbestückung.

Vorteilhafterweise sind die Bandschieber durch Aufbringen einer Reibkraft mit dem Trägerband kraftschlüssig in Eingriff bringbar, wobei die Reibkraft bei der Rückbewegung gegenüber der Vorwärtsbewegung reduziert oder aufgehoben ist, so dass ein Bandschlupf möglich ist.

Eine weitere vorteilhafte Ausgestaltung sieht vor, dass die Bandschieber zumindest bei der Vorwärtsbewegung durch eine Feder, insbesondere eine Blattfeder, eine Spiralfeder oder eine Elastomerfeder gegen das Trägerband andrückbar sind, um einen definierten Bandeingriff aufrechtzuerhalten.

Eine besonders für ein Handgerät geeignete Konstruktion sieht vor, dass der Vorschubantrieb einen Elektromotor und einen Exzentertrieb zur Umsetzung einer rotatorischen Motorbewegung in eine translatorische Bandschieberbewegung aufweist.

Dies lässt sich vorteilhaft dadurch realisieren, dass der Vorschubantrieb zwei auf einer Motorwelle in Umfangsrichtung um 180° versetzte Exzenter aufweist, an welchen jeweils ein Bandschieber gelagert ist.

Um eine besonders effektive Bewegungsübertragung zu ermöglichen, ist es vorteilhaft, wenn die Bandschieber als Hebelglied ausgebildet sind und mit einem Hebelende hintereinander oder nebeneinander auf das Trägerband andrücken, wobei die Bandschieber mit dem nachlaufenden Bandabschnitt einen spitzen Winkel vorzugsweise zwischen 30° und 60° Grad einschließen sollten.

Eine weitere Optimierung des Bandtransports lässt sich dadurch erreichen, dass die Auslenkung der Bandschieber vorzugsweise nach Maßgabe der Dicke des Trägerbandes und der Bauhöhe der Verbrauchsmittel im Bereich von 0,05 bis 1,0 mm, bevorzugt im Bereich von 0,1 bis 0,5 mm ausgelegt ist.

Weiterhin ist es von Vorteil, wenn die Bandschieber aus einem Flachmaterial, vorzugsweise durch Ätzen aus einem Metallblech gebildet sind und eine freie Vorderkante für den Bandeingriff aufweisen.

Um den Bandtransport mit geringem Energieaufwand zuverlässig zu bewerkstelligen, ist es günstig, wenn das Trägerband zumindest im Eingriffsbereich der Bandschieber durch eine gegebenenfalls mit einer reibarmen Beschichtung versehene Gleitführung abgestützt ist.

Um ein ungewolltes Mitnehmen des Bandes in Rückwärtsrichtung zu vermeiden, ist es vorteilhaft, wenn die Gleitführung eine rinnenförmige Hohlkontur mit einem Krümmungsradius im Bereich des 0,1- bis 20-fachen, bevorzugt des 0,5- bis 4-fachen der Breite des Trägerbandes aufweist.

Denkbar ist es auch, dass die Bandschieber jeweils einen an einer Kontaktfläche flächig auf das Trägerband aufsetzbaren Schieberfuß aufweisen. In diesem Fall sollte der Schieberfuß mindestens so lang wie die Auslenkung des Bandschiebers in Bandlaufrichtung sein. Dabei lässt sich eine definierte Bandmitnahme dadurch sicherstellen, dass die Kontaktfläche mit scharfkantigen Elementen oder Saugnapfstrukturen oder einer Haftschicht ausgerüstet ist.

Eine weitere baulich vorteilhafte Ausgestaltung sieht vor, dass der Bandspeicher eine Hohlkammer aufweist, die mit einer an die Kontur des Trägerbandes und gegebenenfalls der Verbrauchsmittel angepassten Einführöffnung zum freien Einschieben des Trägerbandes versehen ist, so dass auf zusätzliche Bauelemente und insbesondere einen Wickelkern verzichtet werden kann. Zugleich ist hier durch den Einsatz von Bandschiebern gewährleistet, dass das Band schlaufenfrei bis dicht an die Einführöffnung herangeführt werden kann. Um das Band möglichst effizient und kompakt aufnehmen zu können, ist es vorteilhaft, wenn die Hohlkammer eine lichte Weite aufweist, die unter Freihaltung eines Toleranzbereichs größer als die Breite des Trägerbandes ist, und wenn die Hohlkammer eine lichte Höhe aufweist, die in einem senkrechten Abstand von der Einführöffnung von mindestens dem 200-fachen, bevorzugt dem 1000-fachen der Dicke des Trägerbandes maximal ist.

Im Folgenden wird die Erfindung anhand der in der Zeichnung schematisch dargestellten Ausführungsbeispiele näher erläutert. Es zeigen
- Fig. 1: eine schaubildliche Darstellung eines Testbandsystems zur Blutzuckermessung mit einem oszillierenden Vorschubantrieb;
- Fig. 2: eine ausschnittsweise Draufsicht auf den Vorschubantrieb;
- Fig. 3: eine abgebrochene Seitenansicht eines Bandschiebers mit abgekröpftem Fuß;
- Fig. 4 bis 6: verschiedene Stellungen des Vorschubantriebs in einer Fig. 1 entsprechenden Darstellung.

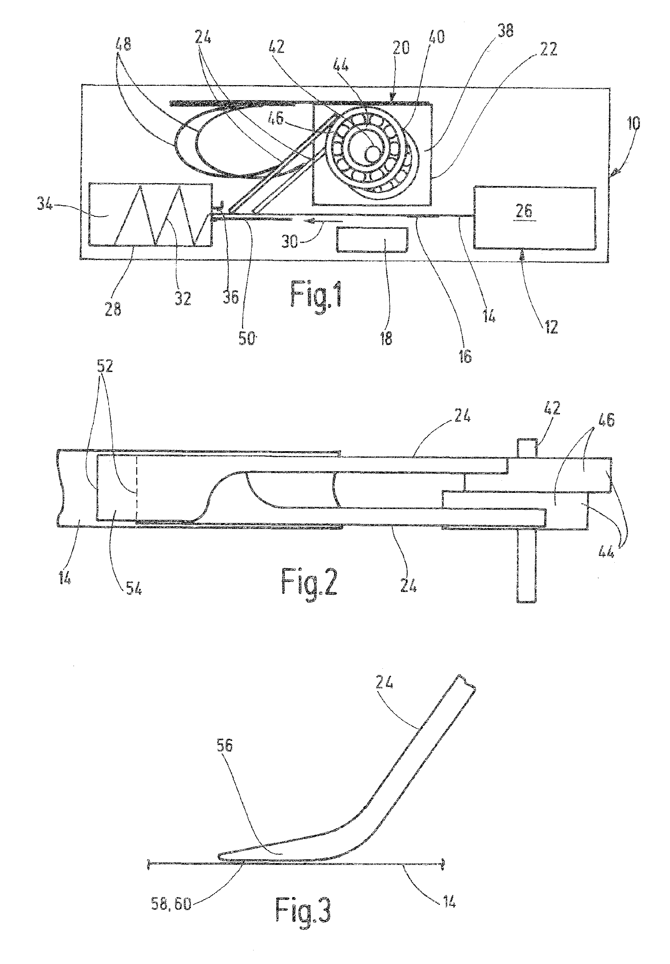

Das in Fig. 1 dargestellte Testbandsystem lässt sich als mobiles Analysegerät 10 mit auswechselbarer Testbandeinheit 12 von einem Probanden für Blutzuckeranalysen vor Ort einsetzen. Es umfasst zu diesem Zweck ein in der Testbandeinheit 12 enthaltenes Trägerband 14, das mit einer Vielzahl von trockenchemischen Testfeldern 16 bestückt ist, die sich für jeweils einen Einmaltest mit einer Blutprobe beaufschlagen und mittels einer photometrischen Messeinheit 18 abtasten lassen. Um die Testfelder 16 in einer Applikations- bzw. Messposition positionieren und nach Gebrauch entsorgen zu können, ist eine ohne Aufwickelspule kontinuierlich arbeitende Bandtransportvorrichtung 20 vorgesehen, die einen oszillierenden Vorschubantrieb 22 für zwei wechselweise wirksame gefederte Bandschieber 24 aufweist.

Wie in Fig. 1 veranschaulicht, kann die Testbandeinheit 12 ein Vorratsbehältnis 26 für unverbrauchtes Bandmaterial und einen Bandspeicher 28 für den in Richtung des Pfeils 30 transportierten verbrauchten Bandabschnitt 32 aufweisen. Alternativ oder ergänzend zu den Testfeldern 16 können auch nicht gezeigte Stechelemente als Verbrauchsmittel auf dem Trägerband 14 angeordnet sein. Als "gebraucht" gilt ein Verbrauchsmittel dann, wenn es seine bestimmungsgemäße Funktion bereits mindestens einmal erfüllt oder den mit der bestimmungsgemäßen Funktion verbundenen Ablauf z.B. wegen Probenmangel oder Bedienfehlern erfolglos durchlaufen hat.

Das Band 14 mit ungebrauchten Verbrauchsmitteln kann aus dem Vorratsbehältnis 26 von einer Rolle, einem wickel oder einem Faltstapel (Leporello) abgezogen werden. Zweckmäßig wird der nachstehend näher erläuterte Vorschubantrieb 22 mit einem einfachen Bandspeicher 28 kombiniert, in den das Band 14 frei hineingeschoben wird. Ein solcher Bandspeicher 28 kann durch eine Hohlkammer 34 gebildet sein, die mit einem Einführschlitz 36 versehen ist.

Die Hohlkammer 34 ist bevorzugt etwas breiter als das Trägerband 14 und weist prinzipiell eine beliebige Querschnittsform auf, z.B. als Quadrat, Rechteck, Dreieck, Rhombus oder Kreis. Um die Bandaufnahme zu erleichtern, kann die Hohlkammer 34 von dem Einführschlitz 36 weg erweitert sein, so dass die lichte Höhe in einem senkrechten Abstand von dem Einführschlitzes von mindestens dem 200-fachen, bevorzugt dem 1000-fachen der Dicke des Trägerbandes 14 maximal ist. Bevorzugt besteht die Innenwand der Hohlkammer 34 aus einem Material und/oder einer Struktur, welche eine geringe Reibung gegenüber dem Band zeigt. Weiterhin sollte diese Oberfläche in solchem Maß elektrisch leitend ausgerüstet sein, dass sich keine permanenten elektrostatischen Aufladungen ausbilden.

Der in Fig. 1 gezeigte Vorschubantrieb 22 umfasst einen Elektromotor 38 und einen Exzentertrieb 40 zur Umsetzung der rotatorischen Motorbewegung in eine translatorische Bandschieberbewegung. Zu diesem Zweck weist der Exzentertrieb 40 zwei auf der Motorwelle 42 in Umfangsrichtung um 180° versetzte Exzenter 44 aufweist, an welchen jeweils ein Bandschieber 24 über einen kugelgelagerten Lagerring 46 gelagert ist. Alternativ ist auch die Verwendung von Gleitlagern möglich.

Die Bandschieber 24 sind an ihren freien Enden durch Aufbringen einer Reibkraft mit dem Trägerband 14 kraftschlüssig in Eingriff bringbar, wobei die Reibkraft bei der Vorwärtsbewegung gegenüber der Rückbewegung durch jeweils eine vorgespannte Blattfeder 48 erhöht wird. Die Feder kann auch als integraler Bestandteil der Schieber ausgeführt werden. Um auf der davon abgewandten Bandseite ein Widerlager zu bilden, ist das Trägerband 14 zumindest über die Eingriffslänge der Bandschieber 24 durch eine gegebenenfalls mit einer reibarmen Beschichtung versehene Gleitführung 50 abgestützt.

Diese führt bis zu dem Bandspeicher 28 heran bzw. ist daran befestigt. Der Eingriffsbereich der Bandschieber 24 reicht bis nahe an den Einführschlitz 36, so dass das flexible Band auch bei geringer Biegesteifigkeit schlaufenfrei einschiebbar ist.

Wie auch aus Fig. 2 ersichtlich, können die Bandschieber 24 als hebelartige flache Formteile beispielsweise durch Ätzen oder Stanzen aus einem dünnen Metallblech geformt sein. Beim Ätzen aus einem gewalzten Stahlblech entsteht die Vorderkante 52 für den Bandeingriff mit gewünschtem kleinem Kantenradius. Die verbreiterten Endabschnitte 54 der Bandschieber 24 sind an die Breite des Bandes 14 angepasst und dicht hintereinander montiert.

In alternativer Ausführung gemäß Fig. 3 können die beiden Bandschieber 24 auch nebeneinander angeordnet sein. Dabei ist jeder Bandschieber 24 so ausgeführt, dass er das Band 14 mit einem abgekröpften Fuß 56 flächig kontaktiert. Der Fuß 56 ist bevorzugt mindestens so lang wie die maximale Auslenkung bzw. Schrittweite des Schiebers 24 bei der Transportbewegung.

Die Kontaktfläche 58 des Fußes 56 mit dem Band 14 ist so ausgeführt, dass bei Vorwärtsbewegung möglichst hohe Reibung zum Band besteht und die Reibung bei der Rückwärtsbewegung möglichst klein ist. Dazu kann die Kontaktfläche 58 mit scharfkantigen Elementen, z.B. Körnern wie bei einem Schleifpapier versehen werden. Es ist auch möglich, die Kontaktfläche mit einer adhäsiven Schicht 60 auszurüsten, z.B. einem Zweikomponenten-PU-Haftlack, wie er von der Firma Surface Contacts, Saarbrücken, Deutschland erhältlich ist. Alternativ kann auch ein silikonbasierter Haftlack eingesetzt werden. Es ist auch denkbar, die Kontaktfläche 58 mit biomimetischen Mikrostrukturen beispielsweise analog dem haftfähigen Fuß eines Gecko umzusetzen.

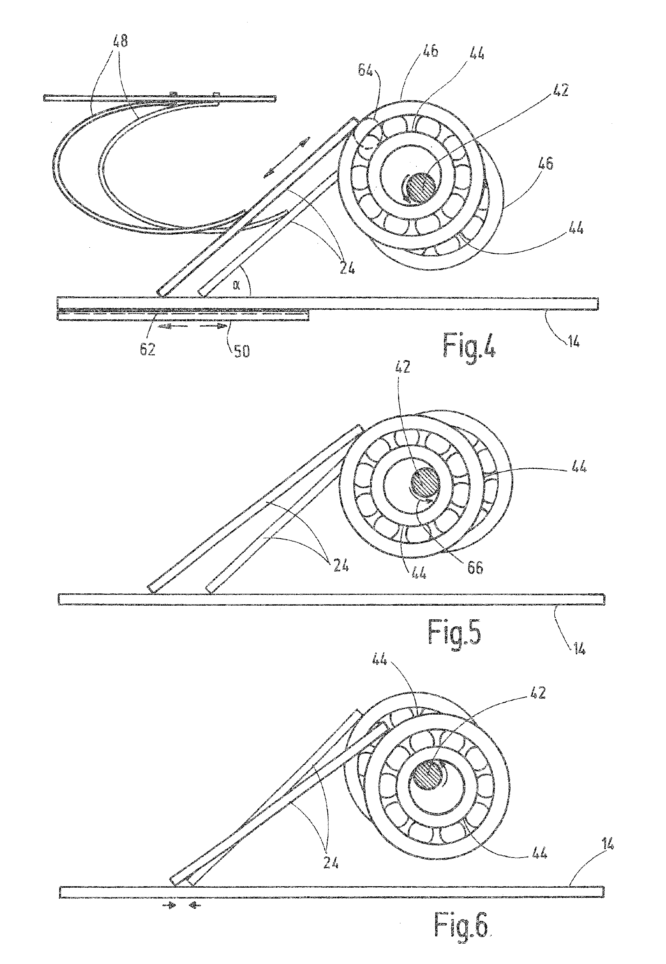

Wie am besten in der Ausschnittsvergrößerung gemäß Fig. 4 ersichtlich, sind die Bandschieber 24 in einem spitzen Winkel α zu dem nachlaufenden bzw. darunterliegenden Bandabschnitt angestellt. Bevorzugt liegt dieser Winkel zwischen 30 und 60 Grad. Bei der Bewegung der Bandschieber 24 ändert sich der Winkel α zum Band nur geringfügig. Die Auslenkung der Bandschieber 24 wird abhängig von der Banddicke und gegebenenfalls der Bauhöhe der Verbrauchsmittel ausgelegt. Bei der bevorzugten Banddicke von 12 *µ*m und einer Testfeld-Bauhöhe von 160 *µ*m liegt die Auslenkung zweckmäßig im Bereich von 0,05 bis 1,0 mm, bevorzugt im Bereich von 0,1 bis 0,5 mm.

Ein ungewolltes Mitnehmen des Trägerbandes 14 mit dem vorderen Schieber 24 bei dessen Rückwärtsbewegung kann durch eine rinnenförmige Vertiefung 62 der Gleitführung 50 erschwert werden. Deren Radius sollte im Bereich des 0,1- bis 20-fachen, bevorzugt des 0,5- bis 4-fachen der Breite des Trägerbandes 14 liegen. Entsprechend werden die Vorderkanten 52 der Schieber passend elliptisch ausgeführt. Auch andere, aber jeweils zueinander komplementäre Ausgestaltungen sind denkbar und können den gewünschten Zweck erfüllen.

Die Motorwelle 42 ist drehfest mit den Exzentern 44 verbunden, während die Lagerringe 46 weitgehend reibungsfrei auf den Exzentern 44 drehbar sind. Durch die exzentrische Bewegung erfahren die an den Lagerringen 46 befestigten Bandschieber antriebsseitig eine Kreisbewegung 64 mit einem Radius, der gleich der Exzentrizität des zugehörigen Exzenters 44 ist. Abtriebsseitig wird diese Kreisbewegung an den Vorderkanten 52 in eine vor und zurück gehende Bewegung umgesetzt, die bei den beiden Schiebern um 180° versetzt ist.

Der Ablauf des Bandtransportes unter Drehung der Motorwelle in Richtung des Pfeils 66 wird in der Abfolge der Fig. 4 bis 6 für einen Drehwinkel von 0°, 60° und 180° weiter veranschaulicht. Zunächst bewegt sich der in Bandlaufrichtung 30 gesehen vordere Schieber 24 entgegen der Kraft seiner Andrückfeder 48 nach vorn und nimmt dabei das Band 14 mit. Gleichzeitig bewegt sich der hintere Schieber 24 rückwärts und gleitet aufgrund der dabei reduzierten Andrückkraft der Feder 48 mit Schlupf über das Band 14. Bei einem Drehwinkel von 60° gemäß Fig. 5 haben die Vorderkanten 52 der beiden Schieber einen maximalen Abstand voneinander und die Bewegungsrichtungen kehren sich um. Wie in Fig. 6 für den Drehwinkel von 180° gezeigt, bewegt sich nun der hintere Schieber nach vorn und transportiert das Band mit, während der vordere Schieber rückwärts gleitet. Die Drehbewegung der Motorwelle 42 wird somit über die oszillierende Schieberbewegung des Vorschubantriebs 22 in einen kontinuierlichen Bandtransport umgesetzt.

## Patentansprüche

1. Bandsystem zur Probenuntersuchung, insbesondere von Körperflüssigkeiten in einem Handgerät, mit einem vorzugsweise als Stech- und/oder Nachweiselemente ausgebildete Verbrauchsmittel (16) tragenden Trägerband (14), einem Bandspeicher (12) zum Aufnehmen des Trägerbandes (14) und einer Transportvorrichtung (20) zum sukzessiven Bereitstellen der Verbrauchsmittel (16) unter Vorschub des Trägerbandes (14) in einer Bandlaufrichtung, **dadurch gekennzeichnet, dass** die Transportvorrichtung (20) zwei mit dem Trägerband (14) in Eingriff bringbare Bandschieber (24) aufweist, und dass die Bandschieber (24) durch einen oszillierenden Vorschubantrieb (22) wechselweise in Bandlaufrichtung vorwärts und zurück bewegbar sind.

2. Bandsystem nach Anspruch 1, **dadurch gekennzeichnet, dass** die Bandschieber (24) durch Aufbringen einer Reibkraft mit dem Trägerband (14) in Eingriff bringbar sind, wobei die Reibkraft bei der Rückbewegung gegenüber der Vorwärtsbewegung reduziert oder aufgehoben ist.

3. Bandsystem nach Anspruch 1 oder 2, **dadurch gekennzeichnet, dass** die Bandschieber (24) zumindest bei der Vorwärtsbewegung durch eine Feder (48), insbesondere eine Blattfeder, eine Spiralfeder oder eine Elastomerfeder gegen das Trägerband (14) andrückbar sind.

4. Bandsystem nach einem der Ansprüche 1 bis 3, **dadurch gekennzeichnet, dass** der Vorschubantrieb (22) einen Elektromotor (38) und einen Exzentertrieb (40) zur Umsetzung einer rotatorischen Motorbewegung in eine translatorische Bandschieberbewegung aufweist.

5. Bandsystem nach einem der Ansprüche 1 bis 4, **dadurch gekennzeichnet, dass** der Vorschubantrieb (22) zwei auf einer Motorwelle (42) in Umfangsrichtung um 180° versetzte Exzenter (44) aufweist, an welchen jeweils ein Bandschieber (24) gelagert ist.

6. Bandsystem nach einem der Ansprüche 1 bis 5, **dadurch gekennzeichnet, dass** die Bandschieber (24) als Hebelglied ausgebildet sind und mit einem Hebelende hintereinander oder nebeneinander auf das Trägerband (14) andrücken, wobei die Bandschieber (24) mit dem nachlaufenden Bandabschnitt einen spitzen Winkel vorzugsweise zwischen 30° und 60° Grad einschließen.

7. Bandsystem nach einem der Ansprüche 1 bis 6, **dadurch gekennzeichnet, dass** die Auslenkung der Bandschieber (24) vorzugsweise nach Maßgabe der Dicke des Trägerbandes (14) und der Bauhöhe der Verbrauchsmittel (16) im Bereich von 0,05 bis 1,0 mm, bevorzugt im Bereich von 0,1 bis 0,5 mm gewählt ist.

8. Bandsystem nach einem der Ansprüche 1 bis 7, **dadurch gekennzeichnet, dass** die Bandschieber (24) aus einem Flachmaterial, vorzugsweise durch Ätzen aus einem Metallblech gebildet sind und eine freie Vorderkante (52) für den Bandeingriff aufweisen.

9. Bandsystem nach einem der Ansprüche 1 bis 8, **dadurch gekennzeichnet, dass** das Trägerband (14) zumindest im Eingriffsbereich der Bandschieber (24) durch eine gegebenenfalls mit einer reibarmen Beschichtung versehene Gleitführung (50) abgestützt ist.

10. Bandsystem nach Anspruch 9, **dadurch gekennzeichnet, dass** die Gleitführung (50) eine rinnenförmige Hohlkontur (62) mit einem Krümmungsradius im Bereich des 0,1- bis 20-fachen, bevorzugt des 0,5-bis 4-fachen der Breite des Trägerbandes (14) aufweist.

11. Bandsystem nach einem der Ansprüche 1 bis 10, **dadurch gekennzeichnet, dass** die Bandschieber (24) jeweils einen an einer Kontaktfläche (58) flächig auf das Trägerband (14) aufsetzbaren Schieberfuß (56) aufweisen.

12. Bandsystem nach Anspruch 11, **dadurch gekennzeichnet, dass** der Schieberfuß (56) bevorzugt mindestens so lang wie die Auslenkung des Bandschiebers (24) in Bandlaufrichtung ist.

13. Bandsystem nach Anspruch 11 oder 12, **dadurch gekennzeichnet, dass** die Kontaktfläche (58) mit scharfkantigen Elementen oder einer Haftschicht (60) ausgerüstet ist.

14. Bandsystem nach einem der Ansprüche 1 bis 13, **dadurch gekennzeichnet, dass** der Bandspeicher (12) eine Hohlkammer (34) aufweist, die mit einer an die Kontur des Trägerbandes (14) und gegebenenfalls der Verbrauchsmittel (16) angepassten Einführöffnung (36) zum freien Einschieben des Trägerbandes (14) versehen ist.

15. Bandsystem nach Anspruch 14, **dadurch gekennzeichnet, dass** die Hohlkammer (34) eine lichte Weite aufweist, die unter Freihaltung eines Toleranzbereichs größer als die Breite des Trägerbandes (14) ist, und dass die Hohlkammer (34) eine lichte Höhe aufweist, die in einem senkrechten Abstand von der Einführöffnung von mindestens dem 200-fachen, bevorzugt dem 1000-fachen der Dicke des Trägerbandes (14) maximal ist.
